# EUROPEAN PATENT APPLICATION

(11) **EP 3 698 727 A2**
(43) Date of publication of application: **26.08.2020**
(21) Application number: 20158696.3
(22) Date of filing: 21.02.2020
(51) Int. Cl.: A61B 17/115, A61B 90/90, H01R 13/52

(54) **ENCAPSULATED PLUG ASSEMBLY FOR ELECTROMECHANICAL SURGICAL DEVICES**

(30) Priority: 22.02.2019 US 201962808917 P; 27.01.2020 US 202016773197
(71) Applicant: Covidien LP, Mansfield, MA 02048 (US)
(72) Inventor: SGROI, Anthony, Wallingford, CT Connecticut 06492 (US)
(74) Representative: Maschio, Antonio

(57) **Abstract**

A plug assembly for an electromechanical surgical system includes: a housing defining a proximal facing bore; a pair of electrical contacts disposed within the housing, each electrical contact including a distal end portion projecting distally from a distal end of the housing; and a proximal end portion disposed within the proximal facing bore of the housing; a ribbon cable having a distal end portion electrically connected to the proximal end portion of each of the pair of electrical contacts, and being disposed with the proximal facing bore of the housing; and an encapsulating material filling the proximal facing bore of the housing.

## Description

### CROSS-REFERENCE TO RELATED APPLICATIONS

This application claims the benefit of and priority to U.S. Provisional Patent Application Serial No. 62/808,917 filed February 22, 2019, the entire disclosure of which is incorporated by reference herein.

### BACKGROUND

### 1. Technical Field

The present disclosure relates to surgical devices. More specifically, the present disclosure relates to handheld electromechanical surgical systems for performing surgical procedures having an encapsulated plug assembly therein.

### 2. Background of Related Art

One type of surgical device is a circular clamping, cutting and stapling device. Such a device may be employed in a surgical procedure to reattach rectum portions that were previously transected, or similar procedures. Conventional circular clamping, cutting, and stapling devices include a pistol or linear grip-styled structure having an elongated shaft extending therefrom and a staple cartridge supported on the distal end of the elongated shaft. In this instance, a physician may insert the loading unit portion of the circular stapling device into a rectum of a patient and maneuver the device up the colonic tract of the patient toward the transected rectum portions. The loading unit includes a cartridge assembly having a plurality of staples. Along the proximal portion of the transected colon, an anvil assembly can be purse stringed therein. Alternatively, if desired, the anvil portion can be inserted into the colon through an incision proximal to the transected colon. The anvil and cartridge assemblies are approximated toward one another and staples are ejected from the cartridge assembly toward the anvil assembly thereby forming the staples in tissue to affect an end-to-end anastomosis, and an annular knife is fired to core a portion of the clamped tissue portions. After the end-to-end anastomosis has been effected, the circular stapling device is removed from the surgical site.

A number of surgical device manufacturers have also developed proprietary powered drive systems for operating and/or manipulating the end effectors. The powered drive systems may include a powered handle assembly, which may be reusable, and a disposable end effector that is removably connected to the powered handle assembly.

Many of the existing end effectors for use with existing powered surgical devices and/or handle assemblies are driven by a linear driving force. For example, end effectors for performing endo-gastrointestinal anastomosis procedures, end-to-end anastomosis procedures and transverse anastomosis procedures, are actuated by a linear driving force. As such, these end effectors are not compatible with surgical devices and/or handle assemblies that use rotary motion.

In order to make the linear driven end effectors compatible with powered surgical devices that use a rotary motion to deliver power, a need exists for adapters to interconnect the linear driven end effectors with the powered rotary driven surgical devices. These adapters may also be reusable, and as such, need to able to withstand multiple sterilization cycles.

As these adapters are becoming more sophisticated and include various electronic components, there is a need for electronic components disposed within the adapters that can withstand multiple autoclave cycles. For example, the electronic components may include flex or ribbon cables fabricated out of a material that is highly resistant to the high pH environments of disinfecting chemicals, such as, potassium hydroxide (KOH), and can also resist high temperature autoclave steam and the associated pressures of the autoclave (+atm/-atm). It is also desired that the housing of these electronic components also be fabricated out of a material that is highly resistant to the high PH environments of disinfecting chemicals (KOH) and that can also resist high temperature autoclave steam and the associated pressures of autoclave (+atm/-atm).

### SUMMARY

According to one embodiment of the present disclosure, a plug assembly for an electromechanical surgical system is disclosed. The plug assembly includes: a housing defining a proximal facing bore; a pair of electrical contacts disposed within the housing, each electrical contact including a distal end portion projecting distally from a distal end of the housing; and a proximal end portion disposed within the proximal facing bore of the housing; a ribbon cable having a distal end portion electrically connected to the proximal end portion of each of the pair of electrical contacts, and being disposed with the proximal facing bore of the housing; and an encapsulating material filling the proximal facing bore of the housing.

According to another embodiment of the present disclosure, the plug assembly includes: a housing defining a proximal facing bore, the housing including a proximally extending central rib located within the proximal facing bore; a pair of electrical contacts disposed within the housing, wherein the pair of electrical contacts are spaced apart from one another, each electrical contact including a distal end portion projecting distally from a distal end of the housing; and a proximal end portion disposed within the proximal facing bore of the housing; a ribbon cable having an axially split distal end portion defining a pair of fingers spaced apart from one another by a gap, each finger being electrically connected to the proximal end portion of a respective one of the pair of electrical contacts, and being disposed with the proximal facing bore of the housing, wherein the rib of the housing is disposed within the gap of the ribbon cable; and an encapsulating material filling the proximal facing bore of the housing.

The housing may be at least partially transparent. The housing may be transparent for light or UV curing. The housing may be fabricated from polyphenylsulfone (PPSU) or polysulfone (PSU).

The encapsulating material may be a light or UV curable material. The encapsulating material may be resin or acrylic resin.

The housing may define a distal facing bore therein. The plug assembly may further include a seal member disposed within the distal facing bore of the housing.

The housing and the seal member may form a fluid-tight seal therebetween. The seal member may be fabricated from silicone, rubber, plastic or polymer.

The seal member may include a distal portion projecting distally from the housing, and a proximal portion extending from a side surface of the housing.

The distal end portion of each of the pair of electrical contacts may extend distally beyond the seal member.

The seal member may include at least one circumferential ridge extending therearound.

Each electrical contact may include a nub projecting from the proximal end portion thereof. The distal end portion of the ribbon cable may define a respective solder recess formed therein for receipt of a respective nub.

Each electrical contact may include a pair of nubs projecting from the proximal end portion thereof. The distal end portion of the ribbon cable may define a respective pair of solder recesses formed therein for receipt of a respective pair of nubs.

Each electrical contact may include a nub projecting from the proximal end portion thereof. Each finger of the distal end portion of the ribbon cable may define a respective solder recess formed therein for receipt of a respective nub.

Each electrical contact may include a pair of nubs projecting from the proximal end portion thereof. Each finger of the distal end portion of the ribbon cable may define a respective pair of solder recess formed therein for receipt of a respective pair of nubs.

### BRIEF DESCRIPTION OF THE DRAWINGS

Embodiments of the present disclosure are described herein with reference to the accompanying drawings, wherein:
FIG. 1 is a perspective view of a handheld surgical device, an adapter assembly, an end effector having a reload and an anvil assembly according to an embodiment of the present disclosure;
FIG. 2 is a perspective view illustrating a connection of the adapter assembly and the handle assembly of FIG. 1 according to an embodiment of the present disclosure;
FIG. 3 is perspective view of internal components of the handle assembly according to an embodiment of the present disclosure;
FIG. 4 is a perspective view of the adapter assembly of FIG. 1 without the reload according to an embodiment of the present disclosure;
FIG. 5 is a perspective view of a distal end portion of the adapter assembly of FIGS. 1-4, illustrating an electrical assembly thereof, in accordance with an embodiment of the present disclosure;
FIG. 6 is a perspective view of the electrical assembly of the adapter assembly of the present disclosure;
FIG. 7 is an enlarged view of a distal portion of the electrical assembly of FIGS. 5 and 6;
FIG. 8 is a distal, perspective view of a plug assembly of the electrical assembly of FIGS. 1-7, configured for connection to the reload of the handheld surgical device;
FIG. 9 is a cross-section view of the plug assembly of FIG. 8, as taken through 9-9 of FIG. 8;
FIG. 10 is a cross-section view of the plug assembly of FIG. 8, as taken through 10-10 of FIG. 8;
FIG. 11 is a cross-section view of the plug assembly of FIG. 8, as taken through 11-11 of FIG. 8;
FIGS. 12A and 12B are rear and front perspective views, respectively, of the plug assembly of FIG. 8, with a seal removed therefrom;
FIG. 13 is a perspective view of the plug assembly of FIG. 8, illustrating an insertion of a ribbon cable and contacts into a housing, of the plug assembly;
FIG. 14 is a perspective view of the ribbon cable and contacts of the plug assembly of FIG. 8;
FIG. 15 is a rear, perspective view of the plug assembly of FIG. 8;
FIG. 16 is a perspective view of a distal end portion of a ribbon cable of another embodiment of a plug assembly, in accordance with the present disclosure;
FIG. 17 is a perspective view of the distal end portion of the ribbon cable of FIG. 16, shown connected to a pair of electrical contacts of the plug assembly;
FIG. 18 is a rear, perspective view, with parts separated, of the pair of contacts and ribbon cable of FIGS. 16 and 17, and a housing of the plug assembly of FIGS. 16 and 17;
FIG. 19 is a rear, perspective view, with parts assembled, of the pair of contacts and ribbon cable of FIGS. 16 and 17, and a housing of the plug assembly of FIGS. 16 and 17; and
FIG. 20 is a cross-sectional view of the plug assembly, as taken through 20-20 of FIG. 19.

### DETAILED DESCRIPTION OF EMBODIMENTS

Embodiments of the present disclosure are now described in detail with reference to the drawings in which like reference numerals designate identical or corresponding elements in each of the several views. As used herein, the term "clinician" refers to a doctor, a nurse or any other care provider and may include support personnel. Throughout this description, the term "proximal" will refer to the portion of the device or component thereof that is closer to the clinician and the term "distal" will refer to the portion of the device or component thereof that is farther from the clinician. Additionally, in the drawings and in the description that follows, terms such as front, rear, upper, lower, top, bottom, and similar directional terms are used simply for convenience of description and are not intended to limit the disclosure. In the following description, well-known functions or constructions are not described in detail to avoid obscuring the present disclosure in unnecessary detail.

The present disclosure relates to powered surgical devices having electronic sensors for monitoring mechanical strain and forces imparted on components of the powered surgical devices. More particularly, this disclosure relates to load measuring sensors including load sensing devices as well as analog and digital circuitry that are hermetically sealed such that the load sensors are configured to resist harsh environments. In the event that electrical connections of the powered surgical devices are compromised during use, measurement signals output by the sensors of the present disclosure remain unaltered. In addition, the sensors are programmable allowing for adjustments to gain and offset values in order to optimize the measurement signals.

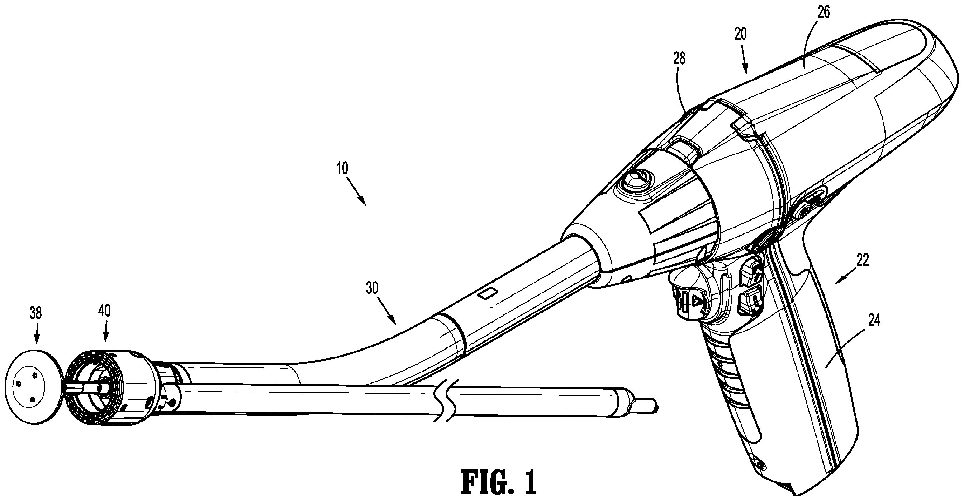

With reference to FIG. 1, a powered surgical device 10 includes a handle assembly 20, which is configured for selective connection with an adapter assembly 30, which in turn, is configured for selective connection with an end effector, such as an annular reload 40. Although generally referred to as being a powered surgical device, it is contemplated that the surgical device 10 may be a manually actuated and may include various configurations.

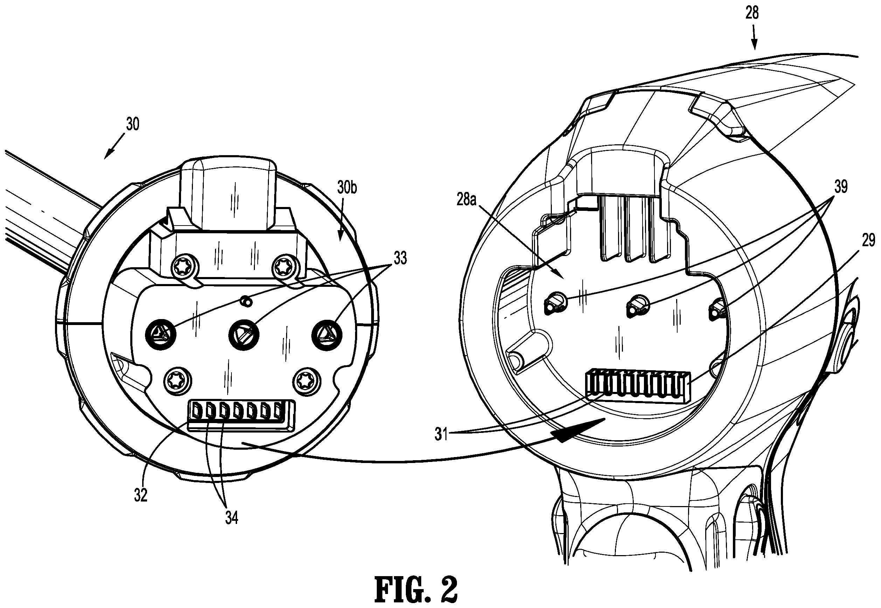

The handle assembly 20 includes a handle housing 22 having a lower housing portion 24, an intermediate housing portion 26 extending from and/or supported on a portion of the lower housing portion 24, and an upper housing portion 28 extending from and/or supported on a portion of the intermediate housing portion 26. As shown in FIG. 2, a distal portion of the upper housing portion 28 defines a nose or connecting portion 28a that is configured to accept a proximal end portion 30b of the adapter assembly 30.

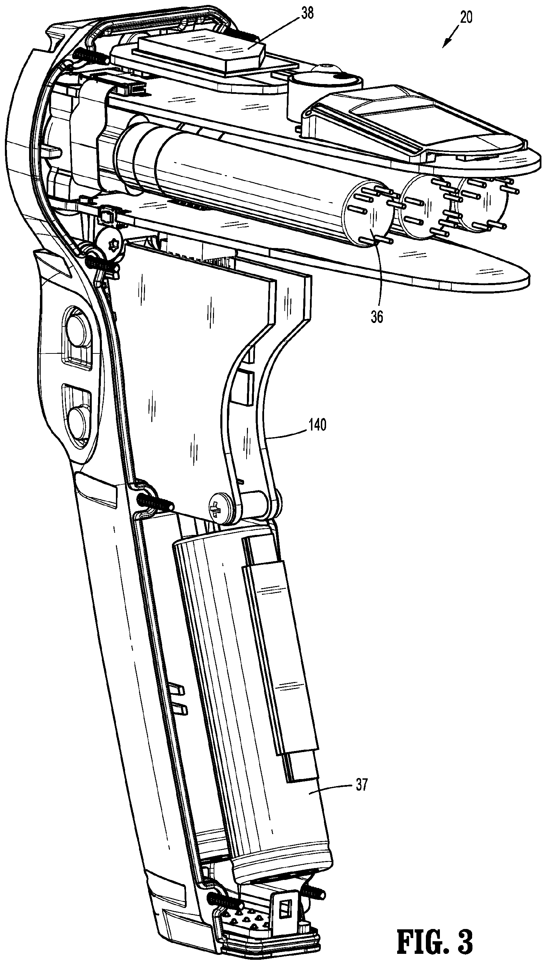

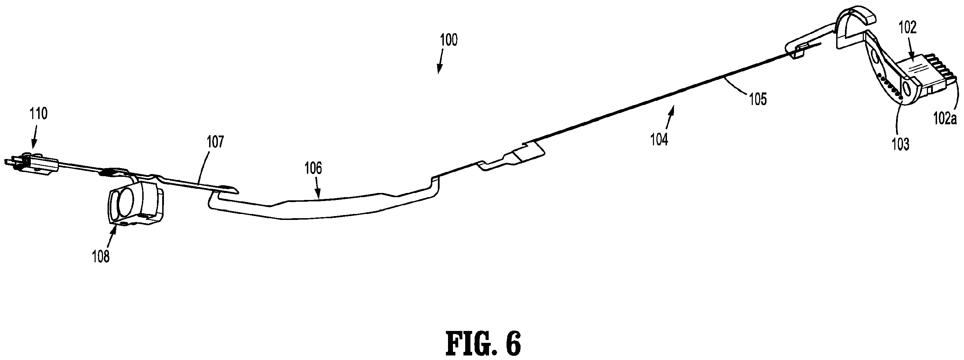

With reference to FIG. 3, the handle assembly 20 includes one or more motors 36 which are coupled to a battery 37. The handle assembly 20 also includes a main controller 38 for operating the motors 36 and other electronic components of the handle assembly 20, the adapter assembly 30, and the reload 40. The motors 36 are coupled to corresponding drive shafts 39 (FIG. 2), which are configured to engage sockets 33 on the proximal end portion 30b, such that rotation of the drive shafts 39 is imparted on the sockets 33. The actuation assembly 52 (FIG. 6B) is coupled to a respective socket 33. The actuation assembly 52 is configured to transfer rotational motion of the sockets 33 into linear motion and to actuate the reload 40 (FIG. 1) along with the anvil assembly 58.

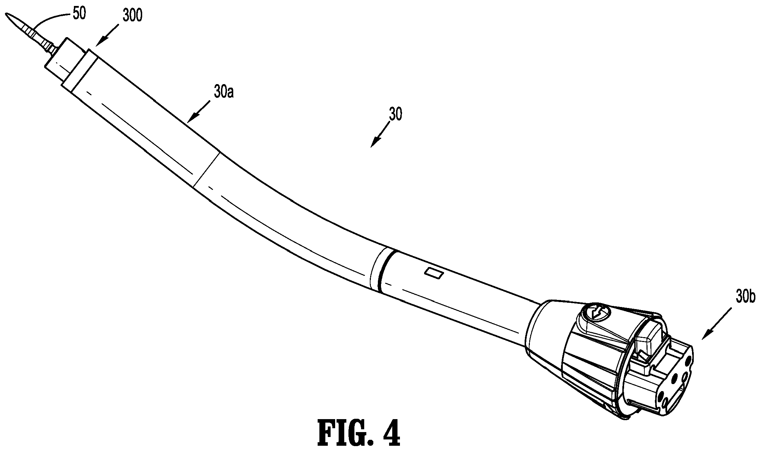

With reference to FIG. 4, the adapter assembly 30 includes a tubular housing 30a that extends between a proximal end portion 30b that is configured for operable connection to the connecting portion 28a of the handle assembly 20 and an opposite, distal end portion 30c that is configured for operable connection to the reload 40. In this manner, the adapter assembly 30 is configured to convert a rotational motion provided by the handle assembly 20 into axial translation useful for advancing/retracting a trocar member 50 slidably disposed within the distal end portion 30c of the adapter assembly 30 for actuating functions of the reload 40, e.g., such as, for firing staples of the reload 40.

With reference to FIG. 2, the connecting portion 28a includes an electrical receptacle 29 having a plurality of electrical contacts 31, which are in electrical communication with electronic (e.g., main controller 38) and electrical components (e.g., battery 37) of the handle assembly 20. The adapter assembly 30 includes a counterpart electrical connector 32 that is configured to engage the electrical receptacle 29. The electrical connector 32 also includes a plurality of electrical contacts 34 that engage and electrically connect to their counterpart electrical contacts 31.

With reference to FIG. 4, the trocar member 50 is slidably disposed within the tubular housing 30a of the adapter assembly 30 and extends past the distal end portion 30c thereof. In this manner, the trocar member 50 is configured for axial translation, which in turn, causes a corresponding axial translation of an anvil assembly 58 (FIG. 1) of the reload 40 to fire the staples (not shown) disposed therein. The trocar member 50 includes a proximal end which is coupled to the tubular housing 30a of the adapter assembly 30. A distal end portion of the trocar member 50 is configured to selectively engage the anvil assembly 58 of the reload 40 (FIG. 4). In this manner, when the anvil assembly 58 is connected to the trocar member 50, as will be described in detail hereinbelow, axial translation of the trocar member 50 in the first direction results in an opening of the anvil assembly 58 relative to the reload 40, and axial translation of the trocar member 50 in a second, opposite direction, results in a closing of the anvil assembly 58 relative to the reload 40.

The reload 40 is configured for operable connection to adapter assembly 30 and is configured to fire and form an annular array of surgical staples, and to sever a ring of tissue.

For a detailed description of an exemplary powered surgical stapler including an adapter assembly and a reload, reference may be made to commonly owned U.S. Patent Application Publication No. 2016/0310134 to Contini et al., titled "Handheld Electromechanical Surgical System," filed April 12, 2016, incorporated in its entirety by reference herein.

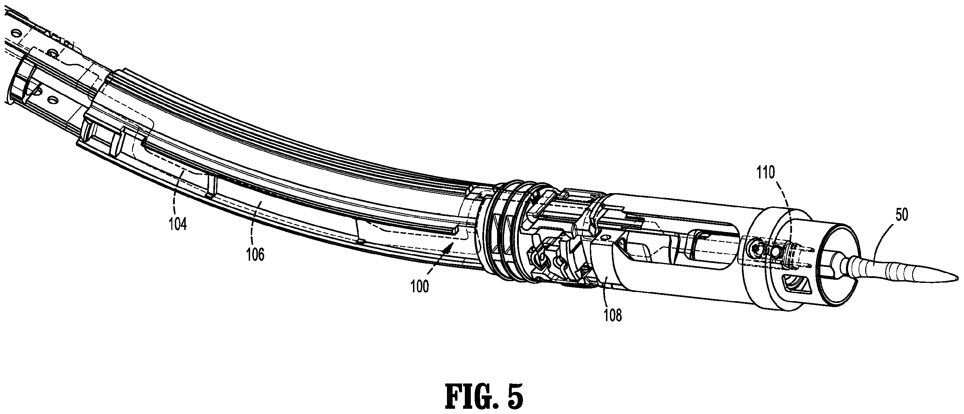

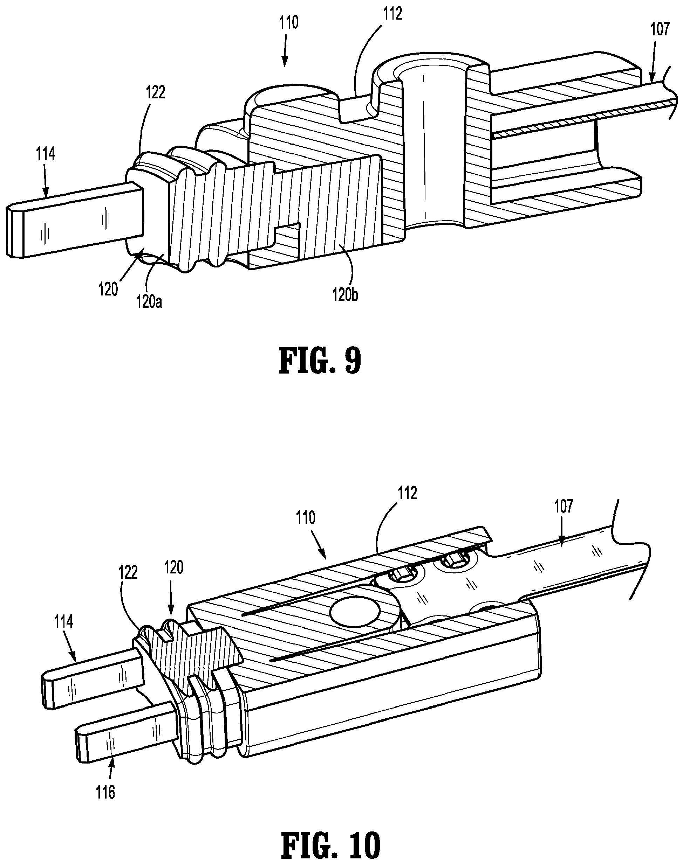

With reference now to FIGS. 5-15, adapter assembly 30 includes an electrical assembly 100 disposed therewithin, and configured for electrical connection with and between handle assembly 20 and reload 40. Electrical assembly 100 provides for communication (e.g., identifying data, life-cycle data, system data, load sense signals) with the main controller 38 of the handle assembly 20 through the electrical receptacle 29 (FIG. 2).

Electrical assembly 100 includes the electrical connector 102; a proximal harness assembly 104, having a ribbon cable 105, connected to electrical connector 102; a distal harness assembly 106, having a ribbon cable 107, connected to proximal harness assembly 104; a load sensing assembly 108 connected to distal harness assembly 106; and a distal electrical plug assembly 110 also connected to distal harness assembly 106. The distal electrical plug assembly 110 is configured to selectively mechanically and electrically connect to a chip assembly (not shown) of reload 40.

Electrical connector 102 of electrical assembly 100 is supported within the proximal end portion 30b of the adapter assembly 30. Electrical connector 102 includes electrical contacts 102a which enable electrical connection to the handle assembly 20. Proximal harness assembly 104 is electrically connected to electrical connector 102 which is disposed on a printed circuit board 103.

The ribbon cable 105, 107 of respective proximal harness assembly 104 and distal harness assembly 106 of electrical assembly 100 includes a body or substrate suitable for supporting and/or electrically connecting electronic components thereto. The substrate of the ribbon cables 105, 107 is formed from one or more layers or sheets of dielectric material, such as a polymer or a ceramic, and one or more layers of conductive material, such as copper foil, that form conductive traces (not explicitly shown) in the substrate. Vias (not shown) may interconnect the conductive traces through different layers of the ribbon cables 105, 107.

In embodiments, the substrate of the ribbon cables 105, 107 is formed from copper-clade polyimides, such as PYRALUX® or NIKAFLEX®, which are registered trademarks owned by DuPont. In some embodiments, the substrate of the ribbon cables 105, 107 is formed from high temperature materials, such as PYRALUX® HT, also a registered trademark owned by DuPont.

In embodiment, it is contemplated that ribbon cables 105, 107 may be fabricated in whole, or in part, from liquid crystal polymer (LCP). LCP is more resistant to high PH environments and autoclave, as compared to ribbon cables without LCP. The ribbon cables 105, 107 may include multiple layers, for example, including a layer of polymide as an inner or outer layer. The multiple layers forming ribbon cables 105, 107 may be bonded using heat bonded lamination (e.g., melting/fusing the layers together) or by using an adhesive layer to bond the layers to one another. In other embodiments, the substrate of the ribbon cables 105, 107 is formed from copper-clade bonded to liquid crystal polymers (LCP) films.

It should be understood that the substrate of the ribbon cables 105, 107 is configured to allow for the fabrication of single or double sided flex circuits, multilayer flex circuits, and rigid flex circuits. The layers of the substrate of the ribbon cables 105, 107 may be joined to one another by, for example, laminating, welding, and/or using adhesives, among other methods and materials within the purview of those skilled in the art.

Plug assembly 110 includes a housing 112 defining a proximal facing bore 112a configured to receive a distal end portion 107a of the ribbon cable 107 of distal harness assembly 106. Electrical contacts or blades 114, 116 are supported within housing 112, with each electrical contact 114, 116 including a respective distal end portion 114a, 116a projecting distally from housing 112. Electrical contacts 114, 116 may be secured within bore 112a of housing 112 in any suitable manner, e.g., press-fit, friction-fit, snap-fit, tacked, welded, potted with a resin material or the like (for fluid-tight retention of electrical contacts 114, 116 within housing 112), glued, etc.

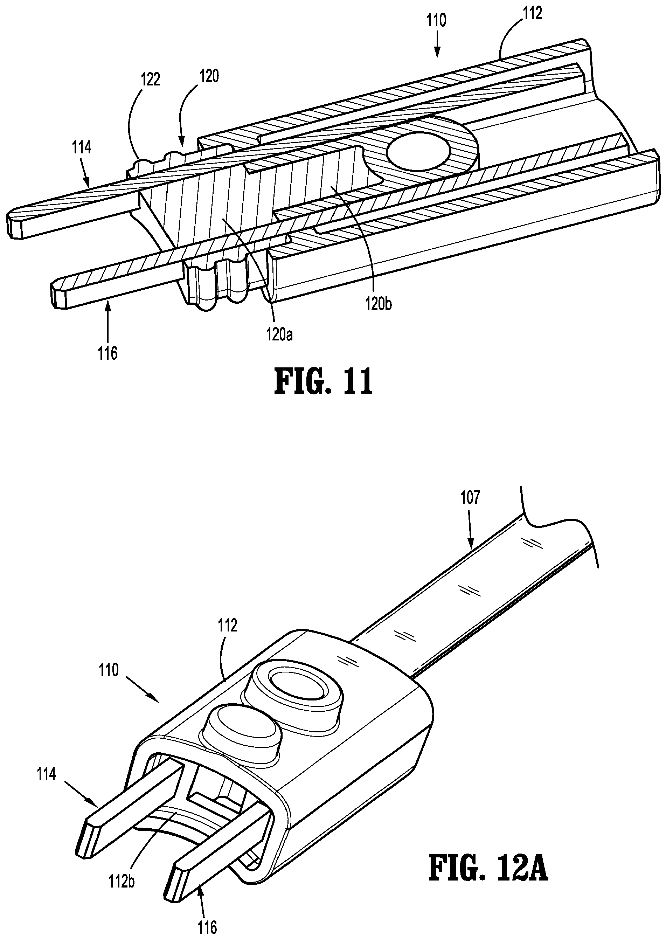

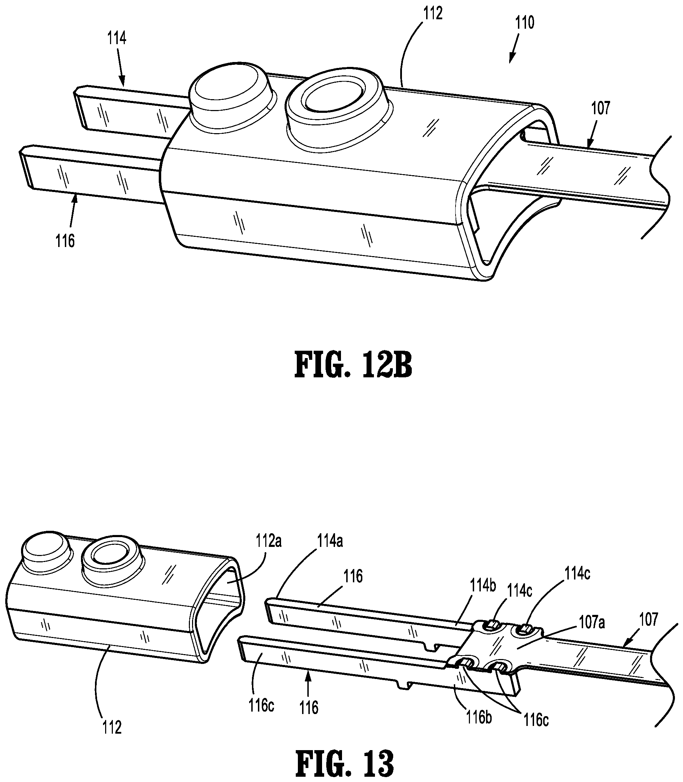

With reference to FIGS. 12A, 12B, 13 and 14, each electrical contact 114, 116 includes a pair of nubs 114c, 116c, respectively, formed in and/or projecting from respective proximal end portions 114b, 116b thereof. The nubs 114c, 116c defines solder areas for electrical connection to a distal end portion 107a of ribbon cable 107. Specifically, distal end portion 107a of ribbon cable 107 includes a first pair of soldering recess 107b₁ formed in a first side edge thereof, and a second pair of soldering recess 107b₂ formed in a second side edge thereof. The first pair of soldering recess 107bi are configured to register with the pair of nubs 114c of electrical contact 114, and the second pair of soldering recess 107b₂ are configured to register with the pair of nubs 116c of electrical contact 116. Each soldering recess of the first pair and the second pair of soldering recess 107b₁, 107b₂ may define solder pads (e.g., castellated type solder pads) for electrical connection with respective nubs 114c, 116c of electrical contacts 114, 116. It is contemplated that the distal end portion 107a of ribbon cable 107 may be soldered or secured to each electrical contact 114, 116 via an immersion tin process, electroless nickel immersion gold (ENIG) process, or the like know by those of skill in the art.

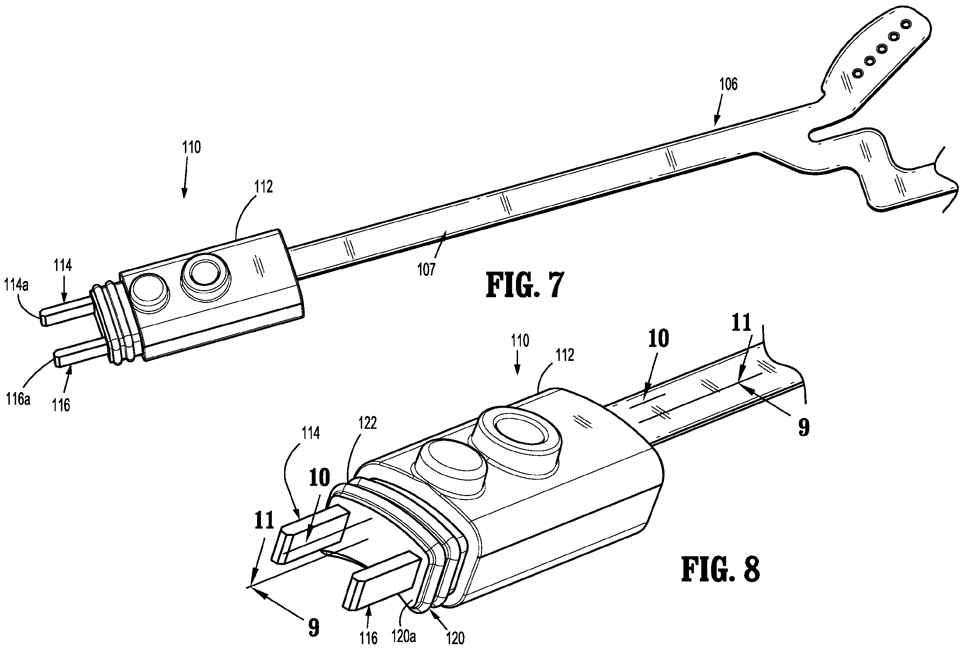

With reference to FIGS. 7-11, plug assembly 110 includes a seal member 120 disposed within a distal facing bore 112b (see FIG. 12A) of housing 112. Seal member 120 includes a pair of slots formed therein for passage of distal end portion 114a, 116a of electrical contacts 114, 116 therethrough. Seal member 120 is secured to a distal end of the housing 112 and includes a distal portion 120a that extends from the distal end of housing 112, and a proximal portion 120b that is configured to be received through at least one side surface of housing 112 and form an interlock therewith. Seal member 120 may include circumferential ridges 122 configured to engage an inner wall of a plug receptacle of reload 40 (not shown) to facilitate a friction fit and fluid-tight seal between plug assembly 110 of adapter assembly 30 and the plug receptacle of reload 40.

Seal member 120 may be formed of silicone, rubber, plastic, polymer, or any other suitable material.

As mentioned above, distal end portion 114a, 116a of electrical contacts 114, 116, respectively, of plug assembly 110, extend through and from seal member 120. The distal end portion 114a, 116a of electrical contacts 114, 116 are configured to electrically couple with respective contact members of a complimentary plug receptacle of reload 40 (not shown).

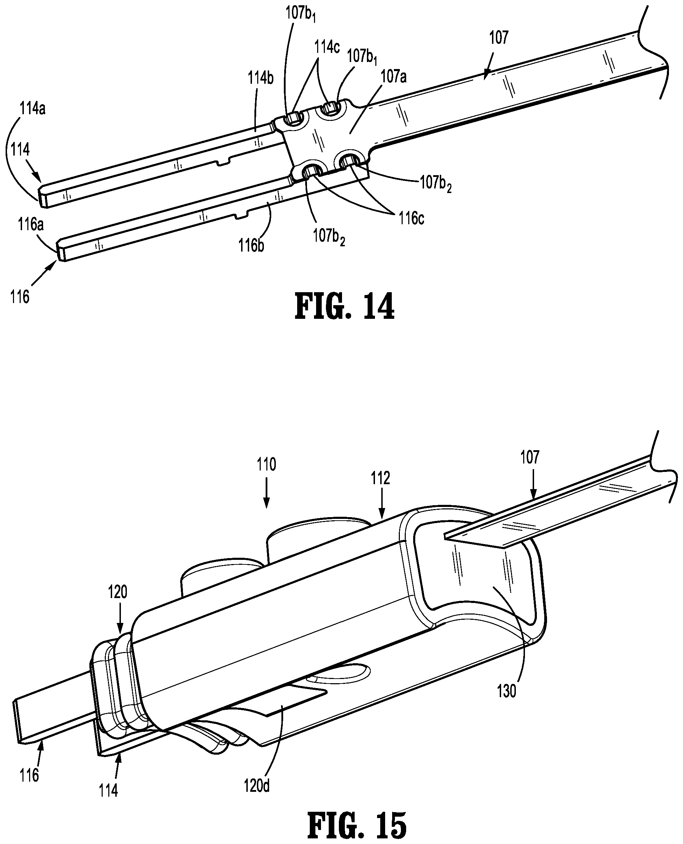

With reference to FIG. 15, with electrical contact 114, 116 and distal end portion 107a of ribbon cable 107 disposed within housing 112, proximal facing bore 112a of housing 112 may be filled with an encapsulating material 130 (e.g., resin, acrylic resin) which is resistant to disinfecting and sterilization operations (e.g., washing, rinsing, autoclaving, etc.).

Housing 112 may be transparent or near transparent, thereby enabling use of encapsulating materials 130 which are light or UV curable. The transparency of the housing 112 allows for the encapsulating material 130 to be cured after full assembly of plug assembly 110. Accordingly, housing 112 may be fabricated from polyphenylsulfone (PPSU) using an injection molding process, extrusion process, or the like, or polysulfone (PSU) which is also transparent for light/UV curing. In an embodiment, housing 112 may be fabricated from opaque materials providing that Room-Temperature-Vulcanizing (RTV) Encapsulates are selected that can cure without the need for UV or light cure processes.

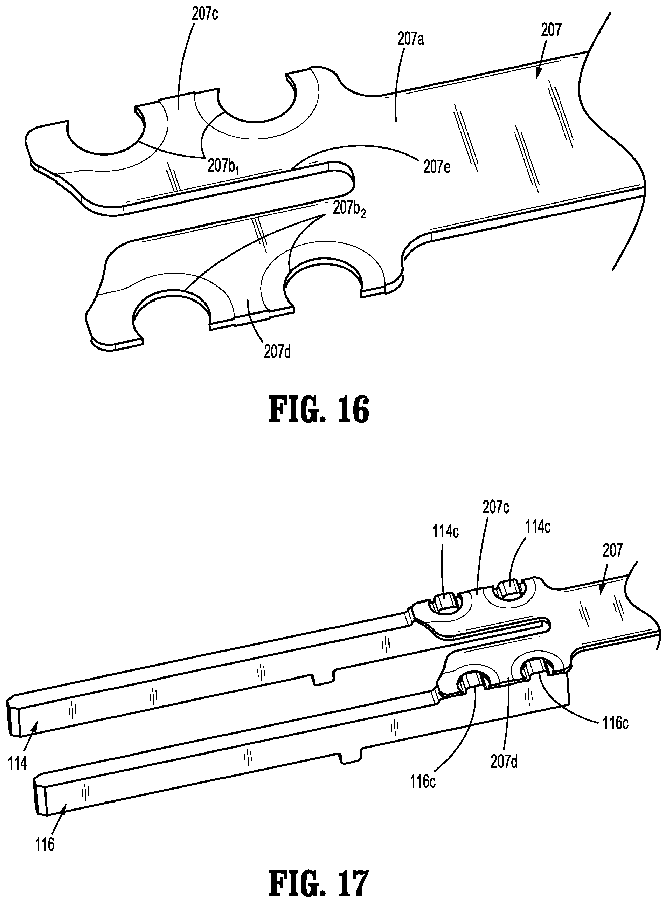

Turning now to FIGS. 16-20, a plug assembly, in accordance with an alternate embodiment of the present disclosure, is generally designated as 210. Plug assembly 210 is substantially similar to plug assembly 110, and in the interest of brevity, only the differences therebetween will be described in detail herein below.

With reference to FIGS. 16-18, a distal end portion 207a of a ribbon cable 207 of an electrical assembly 100, for use with plug assembly 210, is shown and described. Distal end portion 207a of ribbon cable 207 is split, divided or bifurcated to include a pair of distally extending fingers 207c, 207d separated by a gap or space 207e.

Distal end portion 207a of ribbon cable 207 includes a first pair of soldering recess 207bi formed in a first side edge of first finger 207c, and a second pair of soldering recess 207b₂ formed in a second side edge of second finger 207d. The first pair of soldering recess 207b₁ are configured to register with the pair of nubs 114c of electrical contact 114, and the second pair of soldering recess 207b₂ are configured to register with the pair of nubs 116c of electrical contact 116. Each soldering recess of the first pair and the second pair of soldering recess 207b₁, 207b₂ may define solder pads (e.g., castellated type solder pads) for electrical connection with respective nubs 114c, 116c of electrical contacts 114, 116. It is contemplated that first and second fingers 207c, 207d of distal end portion 207a of ribbon cable 207 may be soldered or secured to each electrical contact 114, 116, respectively, via an immersion tin process, electroless nickel immersion gold (ENIG) process, or the like know by those of skill in the art.

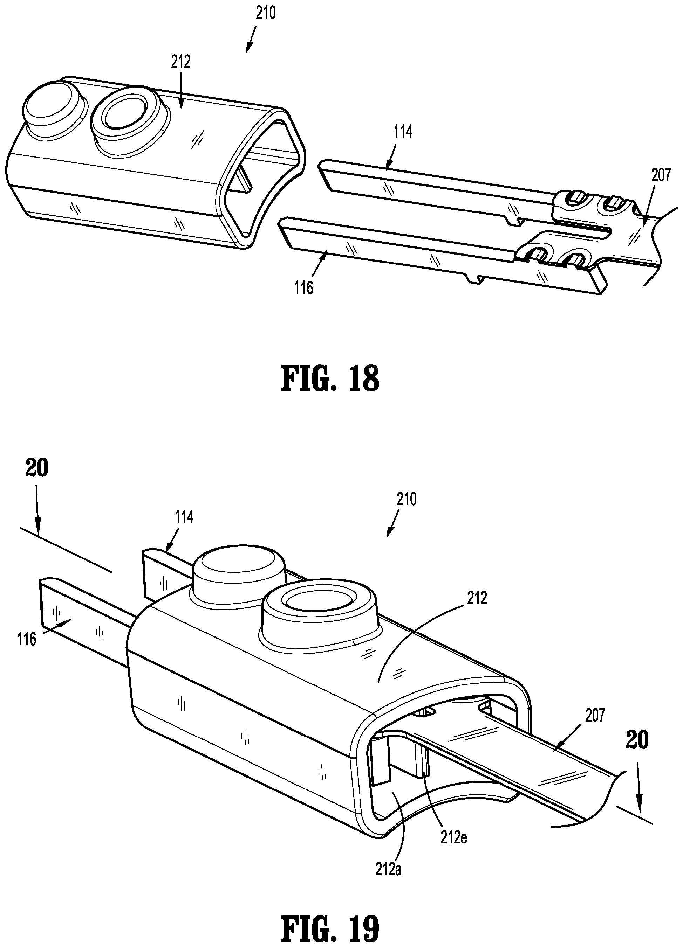

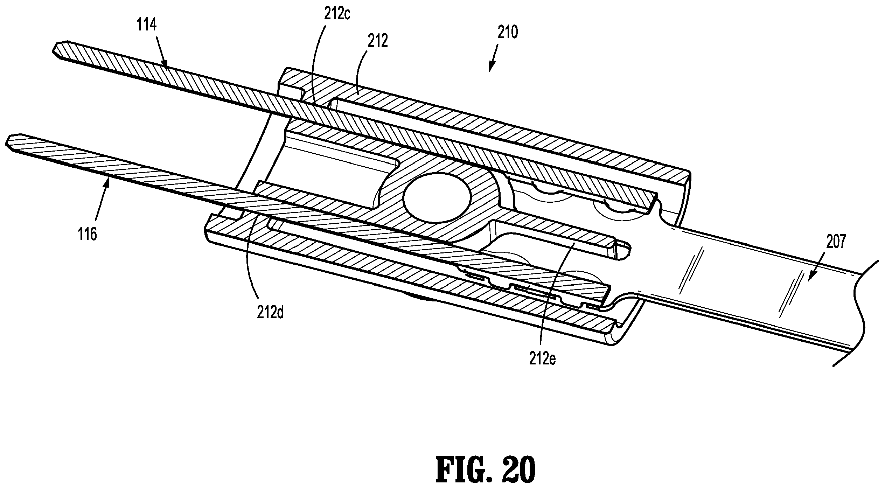

With reference now to FIGS. 18-20, a housing 212 of plug assembly 210, is shown and described. Housing 212 defines a proximal facing bore 212a configured to receive a distal end portion 207a of ribbon cable 207. Electrical contacts or blades 114, 116 are supported within housing 112, with each electrical contact 114, 116 including a respective distal end portion 114a, 116a disposed within respective lumens 212c, 212d (FIG. 20) defined therewithin, and projecting distally from housing 212. Electrical contacts 114, 116 may be secured within bore 212a of housing 212 in any suitable manner, e.g., press-fit, friction-fit, snap-fit, tacked, welded, potted with a resin material or the like (for fluid-tight retention of electrical contacts 114, 116 within housing 212), glued, etc.

Housing 212 includes a central, proximally extending rib or wall 212e disposed within proximal facing bore 212a. Rib 212e is configured and dimensioned to substantially fill gap 207e defines in distal end portion 207a of ribbon cable 207 (as described above), when electrical contacts 114, 116 and distal end portion 207a of ribbon cable 207 are seated within housing 212. Rib 212e may be an integral component of housing 212, and thus, may be constructed from the same conducting resistant material as housing 212. It is contemplated, in accordance with the present disclosure, that rib 212e may work in combination with the encapsulating material 130 (e.g., resin, acrylic resin) of proximal facing bore 212a to resist ingress of moisture into plug assembly 210, and resist shorting of the solder pads defined by the first pair and the second pair of soldering recess 207bi, 207b₂ of distal end portion 207a of ribbon cable 207.

It will be understood that various modifications may be made to the embodiments of the presently disclosed adapter assemblies. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A plug assembly for an electromechanical surgical system, the plug assembly comprising:
   a housing defining a proximal facing bore;
   a pair of electrical contacts disposed within the housing, each electrical contact including:
      a distal end portion projecting distally from a distal end of the housing; and
      a proximal end portion disposed within the proximal facing bore of the housing;
   a ribbon cable having a distal end portion electrically connected to the proximal end portion of each of the pair of electrical contacts, and being disposed with the proximal facing bore of the housing; and
   an encapsulating material filling the proximal facing bore of the housing.
2. The plug assembly according to paragraph 1, wherein the housing is at least partially transparent.
3. The plug assembly according to paragraph 1, wherein the housing is transparent for light or UV curing.
4. The plug assembly according to paragraph 2, wherein the housing is fabricated from polyphenylsulfone (PPSU) or polysulfone (PSU).
5. The plug assembly according to paragraph 3, wherein the encapsulating material is a light or UV curable material.
6. The plug assembly according to paragraph 5, wherein the encapsulating material is resin or acrylic resin.
7. The plug assembly according to paragraph 1, wherein the housing defines a distal facing bore therein; and wherein the plug assembly further comprises:
   a seal member disposed within the distal facing bore of the housing.
8. The plug assembly according to paragraph 7, wherein the housing and the seal member form a fluid-tight seal therebetween.
9. The plug assembly according to paragraph 8, wherein the seal member is fabricated from silicone, rubber, plastic or polymer.
10. The plug assembly according to paragraph 8, wherein the seal member includes a distal portion projecting distally from the housing, and a proximal portion extending from a side surface of the housing.
11. The plug assembly according to paragraph 10, wherein the distal end portion of each of the pair of electrical contacts extends distally beyond the seal member.
12. The plug assembly according to paragraph 11, wherein the seal member includes at least one circumferential ridge extending therearound.
13. The plug assembly according to paragraph 1, wherein each electrical contact includes a nub projecting from the proximal end portion thereof, and wherein the distal end portion of the ribbon cable defines a respective solder recess formed therein for receipt of a respective nub.
14. The plug assembly according to paragraph 1, wherein each electrical contact includes a pair of nubs projecting from the proximal end portion thereof, and wherein the distal end portion of the ribbon cable defines a respective pair of solder recesses formed therein for receipt of a respective pair of nubs.
15. The plug assembly according to paragraph 14, wherein the housing is transparent for light or UV curing.
16. The plug assembly according to paragraph 15, wherein the housing is fabricated from polyphenylsulfone (PPSU) or polysulfone (PSU).
17. The plug assembly according to paragraph 16, wherein the encapsulating material is a light or UV curable material.
18. The plug assembly according to paragraph 17, wherein the housing defines a distal facing bore therein; and wherein the plug assembly further comprises:
   a seal member disposed within the distal facing bore of the housing, wherein the housing and the seal member form a fluid-tight seal therebetween.
19. The plug assembly according to paragraph 18, wherein the seal member includes a distal portion projecting distally from the housing, and a proximal portion extending from a side surface of the housing.
20. The plug assembly according to paragraph 19, wherein the distal end portion of each of the pair of electrical contact extends distally beyond the seal member, and wherein the seal member includes at least one circumferential ridge extending therearound.

## Claims

1. A plug assembly for an electromechanical surgical system, the plug assembly comprising:
a housing defining a proximal facing bore;
a pair of electrical contacts disposed within the housing, each electrical contact including:
a distal end portion projecting distally from a distal end of the housing; and
a proximal end portion disposed within the proximal facing bore of the housing;
a ribbon cable having a distal end portion electrically connected to the proximal end portion of each of the pair of electrical contacts, and being disposed with the proximal facing bore of the housing; and
an encapsulating material filling the proximal facing bore of the housing.

2. The plug assembly according to claim 1, wherein the housing is at least partially transparent.

3. The plug assembly according to claim 1, wherein the housing is transparent for light or UV curing.

4. The plug assembly according to claim 2, wherein the housing is fabricated from polyphenylsulfone (PPSU) or polysulfone (PSU).

5. The plug assembly according to claim 3, wherein the encapsulating material is a light or UV curable material; preferably wherein the encapsulating material is resin or acrylic resin.

6. The plug assembly according to any preceding claim, wherein the housing defines a distal facing bore therein; and wherein the plug assembly further comprises:
a seal member disposed within the distal facing bore of the housing; preferably wherein the housing and the seal member form a fluid-tight seal therebetween.

7. The plug assembly according to claim 6, wherein the seal member is fabricated from silicone, rubber, plastic or polymer.

8. The plug assembly according to claim 7, wherein the seal member includes a distal portion projecting distally from the housing, and a proximal portion extending from a side surface of the housing; preferably wherein the distal end portion of each of the pair of electrical contacts extends distally beyond the seal member; preferably wherein the seal member includes at least one circumferential ridge extending therearound.

9. The plug assembly according to any preceding claim, wherein each electrical contact includes a nub projecting from the proximal end portion thereof, and wherein the distal end portion of the ribbon cable defines a respective solder recess formed therein for receipt of a respective nub.

10. The plug assembly according to any preceding claim, wherein each electrical contact includes a pair of nubs projecting from the proximal end portion thereof, and wherein the distal end portion of the ribbon cable defines a respective pair of solder recesses formed therein for receipt of a respective pair of nubs.

11. The plug assembly according to claim 10, wherein the housing is transparent for light or UV curing.

12. The plug assembly according to claim 11, wherein the housing is fabricated from polyphenylsulfone (PPSU) or polysulfone (PSU).

13. The plug assembly according to claim 12, wherein the encapsulating material is a light or UV curable material.

14. The plug assembly according to claim 13, wherein the housing defines a distal facing bore therein; and wherein the plug assembly further comprises:
a seal member disposed within the distal facing bore of the housing, wherein the housing and the seal member form a fluid-tight seal therebetween.

15. The plug assembly according to claim 14, wherein the seal member includes a distal portion projecting distally from the housing, and a proximal portion extending from a side surface of the housing, preferably wherein the distal end portion of each of the pair of electrical contact extends distally beyond the seal member, and wherein the seal member includes at least one circumferential ridge extending therearound.
